# EUROPEAN PATENT SPECIFICATION

(11) **EP 0 432 745 B1**
(45) Date of publication and mention of the grant of the patent: **21.09.1994**
(21) Application number: 90123868.3
(22) Date of filing: 11.12.1990
(51) Int. Cl.: C07D 307/42

(54) **Process for producing furylpropargylcarbinols**
Verfahren zur Herstellung von Furylpropargylcarbinolen
Procédé de préparation de furylpropargylcarbinols

(30) Priority: 15.12.1989 JP 326505/89; 21.06.1990 JP 164444/90
(43) Date of publication of application: 19.06.1991
(73) Proprietor: SUMITOMO CHEMICAL COMPANY LIMITED, Osaka-shi, Osaka 541 (JP)
(72) Inventor: Oda, Yoshiaki, Toyonaka-shi (JP); Matsuo, Sanshiro, Toyonaka-shi (JP); Saito, Kenji, Hirakata-shi (JP)
(74) Representative: Henkel, Feiler, Hänzel & Partner

(56) References cited:
- EP-A- 0 113 107
- EP-A- 0 214 616
- EP-A- 0 247 589
- EP-A- 0 346 881
- US-A- 3 892 782
- METHODEN DER ORGANISCHEN CHEMIE, vol. v/2A, Georg Thieme Verlag, Stuttgart,1977HOUBEN-WEYL pages 77-105; page 96, table 28; page 99, table 28(3)
- CHEMICAL ABSTRACTS, vol. 109, no. 9, August 29, 1988, Columbus, Ohio, USATASHIRO KAZUO et al. "Process for the preparation of furfuryl alcohols asinter-mediates for drugs, agro- chemicals and perfumes" page 679, column 1,abstract- no. 73 308k
- CHEMICAL ABSTRACTS, vol. 109, no. 9, August 29, 1988, Columbus, Ohio, USATASHIRO KAZUO et al. "Process for the preparation of furfuryl alcohols asinter-mediates for drugs, agrochemi-cals and perfumes" page 679, column 1,abstract- no. 73 309m
- CHEMICAL ABSTRACTS, vol. 69, no. 1, July 1, 1968, Columbus,Ohio, USA SASAKI,TADASHI et al. "Heteroaromaticity. VI. Syntheses of some furan deri- vativescontaining acethy- lenic linkage" page 263, column 1, abstract- no. 2 765b

## Description

### BACKGROUND OF THE INVENTION

### FIELD OF THE INVENTION

The present invention relates to a process for producing furylpropargylcarbinol or a derivative thereof 5 represented by the formula (I): 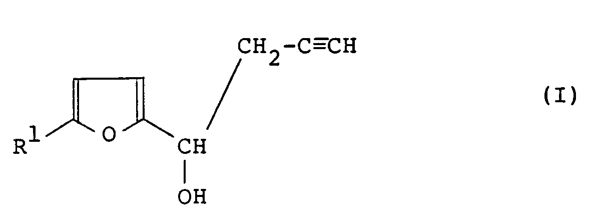
wherein R¹ represents hydrogen or methyl.

The furylpropargylcarbinol and a derivative thereof are very important as intermediates of agricultural chemicals, perfumes and medicines, and particularly 10 applicable to an intermediate of pyrethroid insecticides such as prallethrin.

### DESCRIPTION OF THE PRIOR ART

As a process for producing furylpropargylcarbinol or a derivative thereof, there has heretofore 15 been a known process using the Grignard reaction of propargyl bromide or propargyl chloride with a furfuraltype compound (Japanese Patent Application Kokai No. 59-118780).

Since, however, propargyl bromide and propargyl chloride are detonable or capable of monopropellant-type burning, in view of safety, the inhibition of the detonability is required in the industrial bulk use of them. Therefore, the above-mentioned process is not always an industrially advantageous one.

The present inventors developed a process for producing furylpropargylcarbinol or a derivative thereof by dehydrohalogenation of a haloallylfurylcarbinol or a derivative thereof without using detonable propargyl chloride or propargyl bromide before (EP-89110820.1).

### SUMMARY OF THE INVENTION

The present inventors have found an industrially advantageous process for producing furylpropargylcarbinol or a derivative thereof represented by the formula (I), using neither propargyl chloride nor propargyl bromide.

The present invention relates to a process for producing furylpropargylcarbinol or a derivative thereof represented by the formula (I) which comprises subjecting a haloallylfurylcarbinol or a derivative thereof represented by the formula (II): 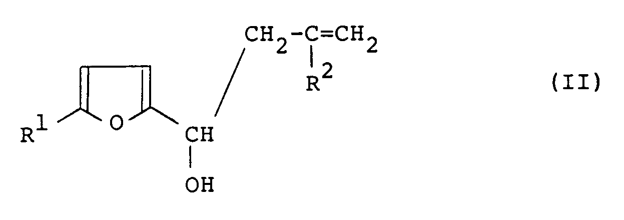
wherein R¹ represents hydrogen or methyl and R² represents chlorine, bromine or iodine, to a dehydrohalogenation reaction in the presence of a diamine with an alkali metal hydroxide or an alkali metal alkoxide.

An object of the present invention is to provide a process for producing furylpropargylcarbinol and derivatives thereof being important as intermediates of agricultural chemicals, perfumes and medicines, and particularly applicable as intermediates of pyrethroid insecticides such as prallethrin.

Another object of the invention is to provide a secure and industrially advantageous process for producing furylpropargylcarbinol and derivatives thereof without using detonable propargyl chloride or propargyl bromide.

### DESCRIPTION OF PREFERRED EMBODIMENTS

The diamine used in the present invention includes aliphatic 1,2- and 1,3-diamines such as 1,2-diaminopropane, 1,3-diaminopropane, ethylenediamine, N,N,N',N'-tetramethylethylenediamine, 2,3-diaminobutane, 1,3-diaminobutane, N,N-dimethylethylenediamine, N,N-dimethyl-1,3-diaminopropane, N,N,N',N'-tetramethyl-1,3-diaminopropane and the like; nonaromatic 1,2- and 1,3-diamines such as 1,5-diazabicyclo[4.3.0]non-5-ene, 1,4-diazabicyclo[2.2.2]octane, 1,8-diazabicyclo[5.4.0]-undec-7-ene, 1,2-diaminocyclohexane, 1,2-diaminocyclopentane and the like; 1,4-diamines such as 1,4-diaminobutane, 1,4-diaminopentane and the like; and 1,6-diamines such as 1,6-diaminohexane, 2,7-diaminooctane and the like. Preferably, aliphatic 1,2- and 1,3-diamines and nonaromatic 1,2- and 1,3-diamines are used. Preferred examples thereof are 1,2-diaminopropane, 1,3-diaminopropane, ethylenediamine, N,N,N',N'-tetramethylethylenediamine, 1,5-diazabicyclo[4.3.0]non-5-ene, 1,4-diazabicyclo[2.2.2]octane, 1,8-diazabicyclo[5.4.0]-undec-7-ene and the like. More preferred examples thereof are 1,2-diaminopropane, 1,3-diaminopropane, ethylenediamine, N,N,N',N'-tetramethylethylenediamine.

The used amount of the diamine is usually 1 to 20 parts by mole, preferably 1 to 15 parts by mole for 1 part by mole of the used haloallylfurylcarbinol or the derivative thereof (II).

The water content of the diamine is usually not more than 10% by weight, preferably not more than 5% by weight.

The base used in the present invention includes alkali metal hydroxides such as sodium hydroxide, potassium hydroxide, lithium hydroxide and the like; alkali metal alkoxides such as sodium methoxide, sodium ethoxide, sodium tert-butoxide, potassium methoxide, potassium ethoxide, potassium tert-butoxide, lithium methoxide, lithium ethoxide, lithium isopropoxide and the like. Preferred examples of the alkali metal hydroxide are sodium hydroxide and potassium hydroxide. Preferred examples of the alkali metal alkoxide are sodium methoxide, sodium ethoxide, potassium methoxide, potassium ethoxide and potassium tert-butoxide.

The used amount of the base is usually 1-10 parts by mole, preferably 1-5 parts by mole for 1 part by mole of the used haloallylfurylcarbinol or the derivative thereof (II).

A solvent such as an ether e.g. tetrahydrofuran, dimethyl ether, 1,4-dioxane or the like; and/or a solvent such as a hydrocarbon e.g. pentane, hexane, benzene, toluene, xylene or the like can be used in the presence of the diamine in the present invention.

The use of a liquid diamine is advantageous from the standpoint of the reaction rate, the volume efficiency (the yield of the target compound per unit volume), the recovery of the diamine and the utilization of the recycled diamine.

If some diamines are deposited as crystals depending upon reaction temperatures in the reaction according to the present invention, those crystals can be dissolved in an organic solvent such as an ether or hydrocarbon mentioned above.

Reaction temperature is usually selected in a range of -20°C to 70°C, preferably 10°C to 50°C.

Reaction time is not particularly critical. The end point of the reaction can be determined by following the rate of disappearance of the haloallylfurylcarbinol or the derivative thereof (II) used as a starting material.

After the reaction is completed, the furylpropargylcarbinol or the derivative thereof (I) can be obtained in an excellent yield from the reaction mixture by, for example, neutralization, filtration, concentration followed by the distillation of the residue.

The haloallylfurylcarbinol or the derivative thereof (II) as a starting material of the present invention can be produced by reacting furfural or a derivative thereof with a 2,3-dihalo-1-propene in the presence of zinc in a solvent.

Thus, according to the present invention, furylpropargylcarbinol and derivatives thereof represented by the formula (I) can be produced in safety and industrially with advance.

The present invention is explained more specifically below referring to Examples, Comparative Example and Referential Examples. However, the present invention should not be construed to be restricted by the Examples.

### Example 1

18.4 g of 2'-chloroallyl-5-methylfurylcarbinol was dissolved in 88.8 g of ethylenediamine of which the water content was 1.4 wt%. The solution was cooled to 10°C. To the solution was added 7.88 g of flaky sodium hydroxide. The reaction mixture was kept at 10°C for 24 hours. The mixture was neutralized with acetic acid, and thereafter, undissolved cakes contained in the solution were filtered off. The filtrate was concentrated under a reduced pressure. The resulting residue was subjected to simple distillation to obtain 13.66 g of 5-methylfurylpropargylcarbinol.
Boiling point: 74°C/0.7 mmHg.
Yield: 92.3%
¹H-NMR data (measurement solvent: CDCl₃, internal standard: TMS, chemical shift: δ-value): 2.03 (t, 1H, J=2.6Hz), 2.25 (d, 3H, J=1.0Hz), 2.70 (dd, 2H, J=6.6Hz, 2.6Hz), 2.89 (brs, 1H), 4.76 (t, 1H, J=6.6Hz), 5.88 (dq, 1H, J=3.3Hz, 10Hz) and 6.17 (d, 1H, J=3.3Hz).

### Example 2

18.2 g of 2'-chloroallyl-5-methylfurylcarbinol was dissolved in 58.6 g of ethylenediamine of which the water content was 5 wt%. To the solution was added 8.19 g of flaky potassium hydroxide. The resulting mixture was kept at 30°C for 5 hours. The mixture was neutralized with acetic acid, and thereafter, undissolved cakes contained in the solution were filtered off. The filtrate was concentrated under a reduced pressure. The resulting residue was subjected to simple distillation to obtain 13.2 g of 5-methylfurylpropargylcarbinol. Yield 90.2%.

### Comparative Example 1

20.0 g of 2'-chloroallyl-5-methylfurylcarbinol was dissolved in 200 g of N,N-dimethylformamide of which the water content was 1 wt%. To the solution was added 12.03 g of flaky potassium hydroxide. The resulting mixture was kept at 50°C for 5 hours. The mixture was neutralized with 10% hydrochloric acid, and thereafter, was concentrated under a reduced pressure. To the resulting residue were added toluene and water, and then extraction and separation was carried out. Thereafter, the organic layer was concentrated and then subjected to simple distillation to obtain 10.35 g of 5-methylfurylpropargylcarbinol. Yield 64.3%.

### Example 3

23.5 g of 2'-bromoallyl-5-methylfurylcarbinol was dissolved in 37.7 g of 1,3-diaminopropane. To the solution was added 8.57 g of flaky potassium hydroxide. The resulting mixture was kept at 30°C for 6 hours. The mixture was neutralized with acetic acid, and thereafter, undissolved cakes contained in the solution were filtered off. The filtrate was concentrated under a reduced pressure. The resulting residue was subjected to simple distillation to obtain 13.6 g of 5-methylfurylpropargylcarbinol. Yield 89.0%.

### Example 4

18.0 g of 2'-chloroallylfurylcarbinol was dissolved in 77.3 g of 1,3-diaminopropane. To the solution was added 6.26 g of flaky sodium hydroxide. The resulting mixture was kept at 20°C for 12 hours. The mixture was neutralized with acetic acid, and thereafter, undissolved cakes contained in the solution were filtered off. The filtrate was concentrated under a reduced pressure. The resulting residue was subjected to simple distillation to obtain 13.1 g of furylpropargylcarbinol.
Boiling point: 76°C/0.8 mmHg
Yield: 92.1%
¹H-NMR data (measurement solvent: CDCl₃, internal standard: TMS, chemical shift: δ-value): 2.06 (t, 1H, J=2.6Hz), 2.72 (brs, 1H), 2.75 (dd, 2H, J=6.3, 2.6Hz), 4.9 (brt, 1H), 6.3 (m, 2H), 7.4 (m, 1H).

### Example 5

15.0 g of 2'-chloroallyl-5-methylfurylcarbinol was dissolved in 122.4 g of 1,8-diazabicyclo[5.4.0]-undec-7-ene. The solution was cooled to 10°C. To the solution was added 4.52 g of flaky sodium hydroxide. The resulting mixture was kept at 10°C for 24 hours. The mixture was neutralized with acetic acid, and thereafter, undissolved cakes contained in the solution were filtered off. The filtrate was concentrated under a reduced pressure. The resulting residue was subjected to simple distillation to obtain 11.0 g of 5-methylfurylpropargylcarbinol. Yield 91.0%.

### Example 6

10.0 g of 2'-chloroallyl-5-methylfurylcarbinol was dissolved in 32.2 g of ethylenediamine of which the water content was 1.4 wt%. The solution was cooled to 10°C. To the solution was added 3.47 g of powdered sodium methoxide. The resulting mixture was kept at 10°C for 20 hours. The mixture was neutralized with acetic acid, and thereafter, undissolved cakes contained in the solution were filtered off. The filtrate was concentrated under a reduced pressure. The resulting residue was subjected to simple distillation to obtain 7.43 g of 5-methylfurylpropargylcarbinol. Yield 92.3%.

### Example 7

18.4 g of 2'-chloroallyl-5-methylfurylcarbinol was dissolved in a mixture of 8.8 g of toluene and 88.8 g of ethylenediamine. The solution was kept at 30°C. To the solution was added 11.2 g of flaky potassium hydroxide. The reaction mixture was kept at 30°C for 17 hours. The mixture was neutralized with acetic acid, and thereafter, undissolved cakes contained in the solution were filtered off. The filtrate was concentrated under a reduced pressure. The resulting residue was subjected to simple distillation to obtain 12.5 g of 5-methylfurylpropargylcarbinol. Yield 83.0%.

### Referential Example 1

Into a reactor were charged 20.0 g of 5-methylfurfural, 88 g of water, 33 g of toluene and 26 g of zinc powder, and the mixture was kept at 33-35°C. Thereinto, 44.4 g of distilled 2,3-dichloro-1-propene was dropped in 20 minutes. The reaction mixture was kept at 33-35°C for 4 hours. After the reaction was completed, the crystals derived from the zinc powder were filtered off. To the filtrate was added 66 g of toluene and the toluene phase was separated. The toluene phase was washed with 30 g of 7%-aqueous sodium carbonate and subsequently with 50 g of water, and thereafter, toluene was distilled off at 60°C or less under a reduced pressure. The resulting residue was subjected to simple distillation to obtain 30.5 g of 2'-chloroallyl-5-methylfurylcarbinol.
Boiling point: 84-85°C/0.8 mmHg.
¹H-NMR data (measurement solvent: CDCl₃, internal standard: TMS, chemical shift: δ-value): 2.27 (s, 3H), 2.75-2.92 (m, 2H), 4.95 (dd, 1H), 5.25 and 5.26 (s, 2H), 5.89 (d, 1H) and 6.13 (d, 1H).

### Referential Example 2

Into the mixture of 11.0 g of 5-methylfurfural, 38.4 g of 3%-aqueous acetic acid, 43.2 g of toluene and 13.1 g of zinc powder, 40.0 g of 2,3-dibromo-1-propene was dropped at 30-35°C in 20 minutes. After the dropping was completed, the reaction mixture was stirred at 30-35°C for 2 hours. After the reaction was completed, the crystals derived from the zinc powder were filtered off and the obtained crystals were washed with 86 g of toluene. The filtrate and the wash liquid were combined to obtain a filtrate mixture. The toluene phase was separated from the filtrate mixture. The toluene phase was washed with 30 g of 7%-aqueous sodium carbonate and subsequently with 50 g of water. Thereafter, toluene was removed at 60°C or less under a reduced pressure. The resulting residue was purified by column chromatography using 100 g of silica gel as a packing and ethyl acetate as an eluent. Thereby, 11.1 g of 2'-bromoallyl-5-methylfurylcarbinol was obtained.
FD-Mass spectrum data: M⁺ 230, M⁺+2 232.
¹H-NMR data (measurement solvent: CDCl₃, internal standard: TMS, chemical shift: δ-value): 2.08 (brs, 1H), 2.28 (d, 3H, J=1.0Hz), 2.93 (m, 2H), 4.98 (dd, 1H, J=5.1, 8.4Hz), 5.54 (d, 1H, J=1.7Hz), 5.72 (d, 1H, J=1.7Hz), 5.91 (m, 1H), 6.16 (d, 1H, J=3.3Hz).
IR data: 3380 cm⁻¹ (O-H, stretching vibration), 1620 cm⁻¹ (vinylidene C=C, stretching vibration). Referential Example 3

The mixture of 18 g of furfural, 33 g of toluene, 88 g of 3%-aqueous acetic acid and 26 g of zinc powder was kept at 33-35°C with stirring.

44.4 g of 2,3-dichloro-1-propene was dropped thereinto at the same temperature in 20 minutes, and thereafter, the reaction mixture was kept at the same temperature for 2 hours. After the reaction was completed, the crystals derived from zinc powder was filtered off. To the filtrate was added 66 g of toluene, and the toluene phase was separated. The toluene phase was washed with 30 g of 7%-aqueous sodium carbonate and subsequently with 50 g of water. Thereafter, toluene was removed at 60°C or less under a reduced pressure. The resulting residue was subjected to simple distillation to obtain 29.4 g of 2-chloroallylfurylcarbinol.
Boiling point: 74°C/0.7 mmHg.
¹H-NMR data (measurement solvent: CDCl₃, internal standard: TMS, chemical shift: δ-value): 2.68 (d, 1H, J=4.3Hz), 2.83 (m, 2H), 5.00 (m, 1H), 5.24 (m, 2H), 6.25 (dd, 1H, J=3.3, 0.7Hz), 6.32 (dd, 1H, J=3.3, 2.0Hz), 7.36 (dd, 1H, J=2.0, 0.7Hz).

## Claims

1. A process for producing furylpropargylcarbinol or a derivative thereof represented by the formula (I): 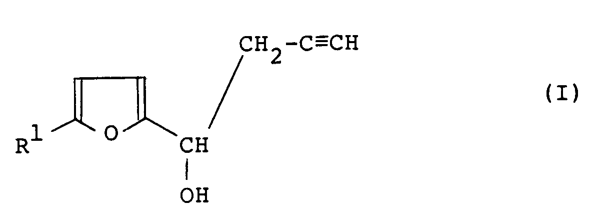 wherein R¹ represents hydrogen or methyl, which comprises subjecting a haloallylfurylcarbinol or a derivative thereof represented by the formula (II): 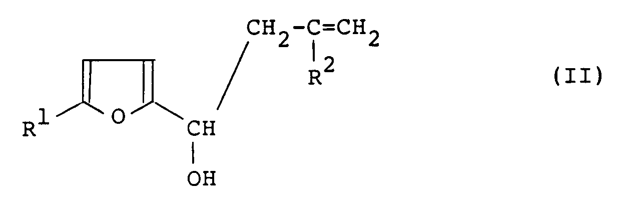 wherein R¹ represents hydrogen or methyl and R² represents chlorine, bromine or iodine, to a dehydrohalogenation reaction in the presence of a diamine with an alkali metal hydroxide.

2. A process for producing furylpropargylcarbinol or a derivative thereof according to Claim 1, wherein the diamine is an aliphatic 1,2- or 1,3-diamine or a nonaromatic 1,2- or 1,3-diamine.

3. A process for producing furylpropargylcarbinol or a derivative thereof according to Claim 2, wherein the aliphatic 1,2- or 1,3-diamine is one selected from the group consisting of 1,2-diaminopropane, 1,3-diaminopropane, ethylenediamine and N,N,N',N'-tetramethylethylenediamine.

4. A process for producing furylpropargylcarbinol or a derivative thereof according to Claim 2, wherein the nonaromatic 1,2- or 1,3-diamine is one selected from the group consisting of 1,5-diazabicyclo[4.3.0]non-5-ene, 1,4-diazabicyclo[2.2.2]octane and 1,8-diazabicyclo-[5.4.0]undec-7-ene.

5. A process for producing furylpropargylcarbinol or a derivative thereof according to any one of Claims 1 to 4, wherein the alkali metal hydroxide is one selected from the group consisting of sodium hydroxide and potassium hydroxide.

6. A process for producing furylpropargylcarbinol or a derivative thereof represented by the formula (I): 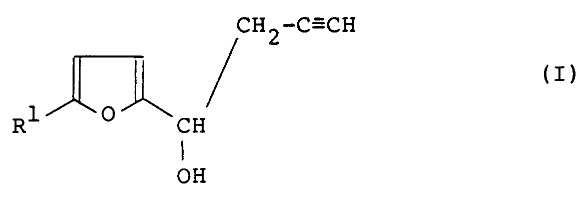 wherein R¹ represents hydrogen or methyl, which comprises subjecting a haloallylfurylcarbinol or a derivative thereof represented by the formula (II): 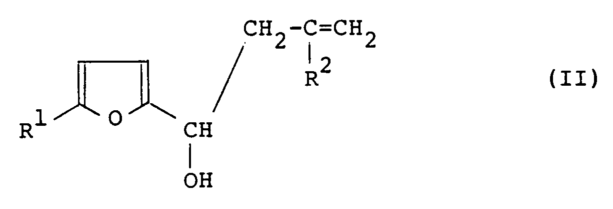 wherein R¹ represents hydrogen or methyl and R² represents chlorine, bromine or iodine, to a dehydrohalogenation reaction in the presence of a diamine with an alkali metal alkoxide.

7. A process for producing furylpropargylcarbinol or a derivative thereof according to Claim 6, wherein the diamine is one selected from the group consisting of 1,2-diaminopropane, 1,3-diaminopropane, ethylenediamine, N,N,N',N'-tetramethylethylenediamine, 1,5-diazabicyclo[4.3.0]non-5-ene, 1,4-diazabicyclo[2.2.2]octane and 1,8-diazabicyclo[5.4.0]undec-7-ene, and the alkali metal alkoxide is one selected from the group consisting of sodium methoxide, sodium ethoxide, potassium methoxide, potassium ethoxide and potassium tert-butoxide.

## Patentansprüche

1. Verfahren zur Herstellung von Furylpropargylcarbinol oder eines Derivats desselben der Formel (I): 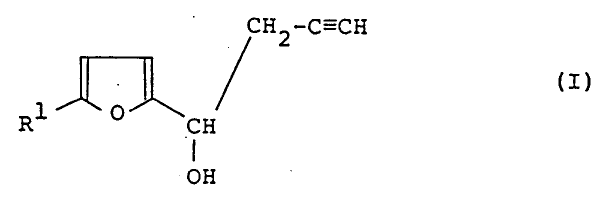 worin R¹ für Wasserstoff oder Methyl steht, durch Dehydrohalogenieren eines Halogenallylfurylcarbinols oder eines Derivats desselben der Formel (II): 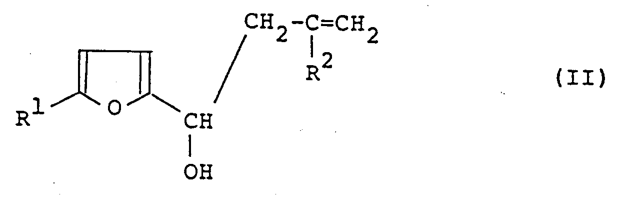 worin R¹ für Wasserstoff oder Methyl steht und R² Chlor, Brom oder Jod bedeutet, in Gegenwart eines Diamins mit einem Alkalimetallhydroxid.

2. Verfahren zur Herstellung von Furylpropargylcarbinol oder eines Derivats desselben nach Anspruch 1, wobei das Diamin aus einem aliphatischen 1,2- oder 1,3-Diamin oder einem nicht-aromatischen 1,2- oder 1,3-Diamin besteht.

3. Verfahren zur Herstellung von Furylpropargylcarbinol oder eines Derivats desselben nach Anspruch 2, wobei das aliphatische 1,2- oder 1,3-Diamin aus einem solchen aus der Gruppe 1,2-Diaminopropan, 1,3-Diaminopropan, Ethylendiamin und N,N,N',N'-Tetramethylethylendiamin ausgewählt ist.

4. Verfahren zur Herstellung von Furylpropargylcarbinol oder eines Derivats desselben nach Anspruch 2, wobei das nicht-aromatische 1,2- oder 1,3-Diamin aus einem solchen aus der Gruppe 1,5-Diazabicyclo[4.3.0]non-5-en, 1,4-Diazabicyclo[2.2.2]octan und 1,8-Diazabicyclo-[5.4.0]undec-7-en ausgewählt ist.

5. Verfahren zur Herstellung von Furylpropargylcarbinol oder eines Derivats desselben nach einem der Ansprüche 1 bis 4, wobei das Alkalimetallhydroxid aus einem solchen aus der Gruppe Natriumhydroxid und Kaliumhydroxid ausgewählt ist.

6. Verfahren zur Herstellung von Furylpropargylcarbinol oder eines Derivats desselben der Formel (I): 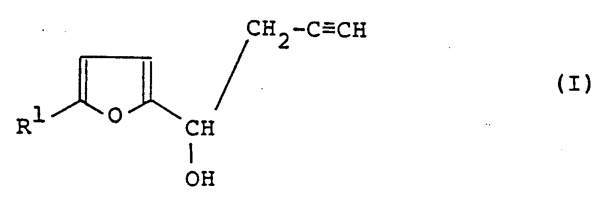 worin R¹ für Wasserstoff oder Methyl steht, durch Dehydrohalogenieren eines Halogenallylfurylcarbinols oder eines Derivats desselben der Formel (II): 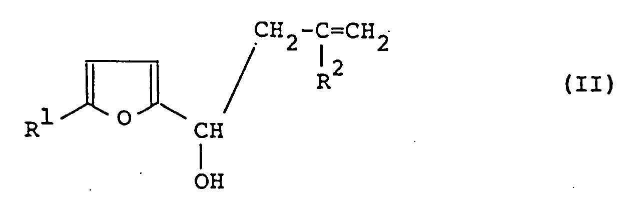 worin R¹ für Wasserstoff oder Methyl steht und R² Chlor, Brom oder Jod bedeutet, in Gegenwart eines Diamins mit einem Alkalimetallalkoxid.

7. Verfahren zur Herstellung von Furylpropargylcarbinol oder eines Derivats desselben nach Anspruch 6, wobei das Diamin aus einem solchen aus der Gruppe 1,2-Diaminopropan, 1,3-Diaminopropan, Ethylendiamin, N,N,N',N'-Tetramethylethylendiamin, 1,5-Diazabicyclo-[4.3.0]non-5-en, 1,4-Diazabicyclo[2.2.2]octan und 1,8-Diazabicyclo[5.4.0]undec-7-en und das Alkalimetallalkoxid aus einem solchen aus der Gruppe Natriummethoxid, Natriumethoxid, Kaliummethoxid, Kaliumethoxid und Kalium-tert.-butoxid ausgewählt sind.

## Revendications

1. Procédé de préparation de furylpropargylcarbinol ou d'un dérivé de celui-ci représenté par la formule (I): 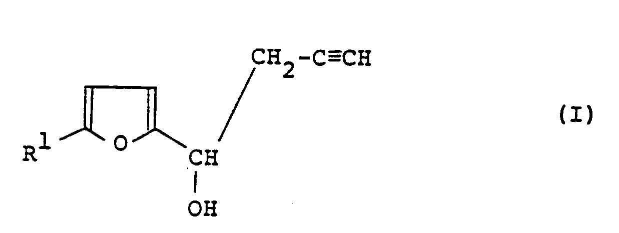 dans laquelle R¹ représente un atome d'hydrogène ou un groupe méthyle, qui comprend une réaction de déshydrohalogénation d'un halogénoallylfurylcarbinol ou d'un dérivé de celui-ci représenté par la formule (II): 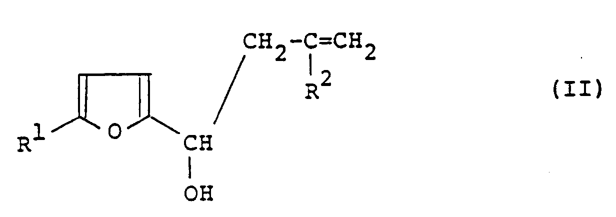 dans laquelle R¹ représente un atome d'hydrogène ou un groupe méthyle et R² représente un atome de chlore, de brome ou d'iode, en présence d'une diamine, avec un hydroxyde de métal alcalin.

2. Procédé de préparation de furylpropargylcarbinol ou d'un dérivé de celui-ci selon la revendication 1, dans lequel la diamine est une 1,2- ou 1,3-diamine aliphatique ou une 1,2- ou 1,3-diamine non aromatique.

3. Procédé de préparation de furylpropargylcarbinol ou d'un dérivé de celui-ci selon la revendication 2, dans lequel la 1,2 ou 1,3-diamine aliphatique est choisie dans le groupe constitué par les 1,2-diaminopropane, 1,3-diaminopropane, éthylènediamine et N,N,N',N'-tétraméthyléthylènediamine.

4. Procédé de préparation de furylpropargylcarbinol ou d'un dérivé de celui-ci selon la revendication 2, dans lequel la 1,2- ou 1,3-diamine non aromatique est choisie dans le groupe constitué par les 1,5-diazabicyclo[4.3.0]none-5-ène, 1,4-diazabicyclo[2.2.2]-octane et 1,8-diazabicyclo[5.4.0]undèc-7-ène.

5. Procédé de préparation de furylpropargylcarbinol ou d'un dérivé de celui-ci selon l'une quelconque des revendications 1 à 4, dans lequel l'hydroxyde de métal alcalin est choisi dans le groupe constitué par l'hydroxyde de sodium et l'hydroxyde de potassium.

6. Procédé de préparation de furylpropargylcarbinol ou d'un dérivé de celui-ci représenté par la formule (I): 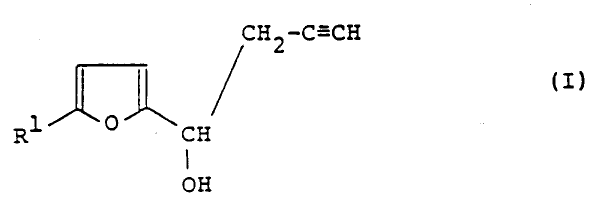 dans laquelle R¹ représente un atome d'hydrogène ou un groupe méthyle, qui comprend une réaction de déshydrohalogénation d'un halogénoallylfurylcarbinol ou d'un dérivé de celui-ci représenté par la formule (II): 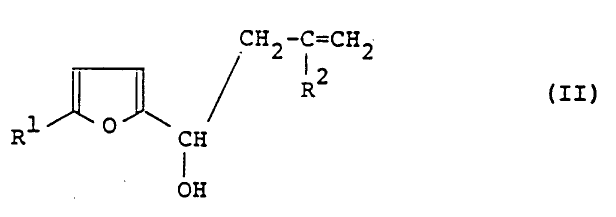 dans laquelle R¹ représente un atome d'hydrogène ou un groupe méthyle et R² représente un atome de chlore, de brome ou d'iode, en présence d'une diamine, avec un alcoolate de métal alcalin.

7. Procédé de préparation de furylpropargylcarbinol ou d'un dérivé de celui-ci selon la revendication 6, dans lequel la diamine est choisie dans le groupe constitué par les 1,2-diaminopropane, 1,3-diaminopropane, éthylènediamine, N,N,N',N'-tétraméthyléthylènediamine, 1,5-diazabicyclo-[4.3.0]none-5-ène, 1,4-diazabicyclo[2.2.2]octane et 1,8-diazabicyclo-[5.4.0]undèc-7-ène, et l'alcoolate de métal alcalin est choisi dans le groupe constitué par les méthanolate de sodium, éthanolate de sodium, méthanolate de potassium, éthanolate de potassium et tert-butanolate de potassium.
